Europäisches Patentamt

European Patent Office

Office européen des brevets

(19)

(11) Numéro de publication: **0 408 922 B1**

(12) **FASCICULE DE BREVET EUROPEEN**

(45) Date de publication de fascicule du brevet: **19.10.94**

(51) Int. Cl.5: **C12N 5/04**, A01N 3/00, A01H 4/00, A01C 1/06

(21) Numéro de dépôt: **90111821.6**

(22) Date de dépôt: **22.06.90**

(54) **Procédé de conservation d'embryons végétaux.**

(30) Priorité: **18.07.89 FR 8909639**

(73) Titulaire: **SOCIETE DES PRODUITS NESTLE S.A.**
**Case postale 353**
**CH-1800 Vevey (CH)**

(43) Date de publication de la demande:
**23.01.91 Bulletin  91/04**

(45) Mention de la délivrance du brevet:
**19.10.94 Bulletin  94/42**

(72) Inventeur: **Florin, Bruno**
**9bis, Rue de la Victoire**
**F-37000 Tours (FR)**
Inventeur: **Petiard, Vincent**
**28, Allée Côte Fleurie**
**F-37100 Tours (FR)**

(84) Etats contractants désignés:
**AT BE CH DE DK ES FR GB GR IT LI LU NL SE**

(56) Documents cités:
**EP-A- 0 136 030**
**EP-A- 0 359 844**
**WO-A-89/05575**

**PLANT CELL REPORTS, vol. 5, no. 5, 1986, pages 372-376, Springer Verlag, Berlin, DE; J.M. AUGEREAU et al.: "Long term storage of callus cultures at low temperatures or under mineral oil layer"**

Rank Xerox (UK) Business Services
(3.10/3.09/3.3.3)

## Description

La présente invention a trait à un procédé de conservation d'embryons végétaux.

De nombreuses espèces de végétaux peuvent être conservées sous forme de suspensions cellulaires, de cals ou de méristèmes.

La conservation d'embryons végétaux se justifie dans de nombreux cas, par exemple pour réguler la production de plantules lorsqu'elle est saisonnière ou pour assurer le maintien d'une lignée clonale.

Le fait de pouvoir conserver des embryons végétaux peut présenter divers avantages: par exemple la possibilité de ralentir temporairement le développement des embryons, le temps nécessaire à leur transport jusqu'au semis ou à leur stockage, ainsi que la possibilité de produire des semences artificielles.

Les embryons somatiques présentent certains avantages pour la multiplication des plantes. Ils proviennent en principe d'une seule cellule et donnent des plantes génétiquement identiques. Les embryons somatiques présentent dès le début de leur formation une structure bipolaire: ils possèdent les deux méristèmes de tige et de racine nécessaires pour produire une plante. L'embryogénèse somatique apparaît donc comme une alternative intéressante pour la propagation des plantes: elle pourrait permettre la multiplication rapide d'espèces ayant un coût de production élevé, ou celle d'individus particulièrement performants provenant de cultures in vitro, ou celle de plantes transformées difficilement manipulables par voie sexuée, par exemple.

Il existe différents procédés connus de conservation de tissus indifférenciés à température réduite et/ou en hypoxie.

D'une manière générale, les tissus sont maintenus sur un milieu semi-solide ou conservés à une température inférieure à 0 ° C.

Un procédé connu consiste à conserver des cals de raisins à une température de 10 ou 15 ° C. Il est possible, afin d'en améliorer la longévité, d'ajouter une couche d'huile de silicone sur les cals, qui restent cependant sur leur substrat nutritif.

Un autre procédé, applicable à des levures ou à des cellules, consiste à former une émulsion aqueuse des cellules avec un milieu huileux, puis à refroidir le tout à une température de l'ordre de -20 ° C/-30 ° C, de telle manière que l'eau reste en surfusion. L'huile sert alors de cryoprotecteur pour les cellules ou levures.

La présente invention a pour but de proposer un procédé de conservation d'embryons végétaux permettant d'assurer un ralentissement notable du développement des embryons isolés de leur milieu de culture pendant un temps déterminé, puis la reprise de ce développement sans apparition d'embryogénèse secondaire.

A cet effet, le procédé selon la présente invention est caractérisé par le fait que les embryons sont maintenus en hypoxie par enrobage avec une couche d'huile, puis les embryons sont refroidis et conservés à une température légèrement supérieure au seuil de sensibilité au froid des embryons considérés.

On a constaté que, de façon étonnante, le maintien en hypoxie sous huile des embryons, associé à une température basse, permettait d'obtenir une inhibition partielle et durable de leur croissance, sans en affecter la viabilité et le pouvoir de germination, tout en maintenant l'intégrité de la structure de l'embryon au cours de son développement ultérieur, sans apparition d'une autre forme de morphogénèse telle que callogénèse ou embryogénèse secondaire, ce tant à la lumière que dans l'obscurité.

En d'autres termes, le présent procédé de conservation d'embryons végétaux permet d'obtenir d'une part la survie des embryons dans leur intégrité morphologique, d'autre part un bon maintien de leur capacité à développer une plantule.

Un avantage de ce procédé est donc de permettre la conservation d'embryons pendant des périodes relativement longues.

Un autre avantage est de permettre d'assurer la conservation à une température facilement accessible (dans un réfrigérateur par exemple) sans avoir à employer de matériel coûteux.

Un autre avantage est de proposer un procédé de conservation rapidement et facilement réalisable, tant à la lumière qu'à l'obscurité.

Les embryons végétaux utilisés dans la présente invention peuvent être de toute origine et de toute espèce telle que la carotte ou le caféier par exemple.

Les embryons peuvent être des embryons somatiques ou des embryons zygotiques.

Les embryons somatiques peuvent être obtenus par l'intermédiaire de suspensions cellulaires indifférenciées. Dans ce cas, des semences de parent d'hybride par exemple peuvent être mises à germer aseptiquement. Les hypocotyles peuvent être coupés puis placés sur un milieu de culture contenant des hormones de croissance. Les cals obtenus peuvent être ensuite dissociés dans un milieu de culture liquide. On obtient alors une suspension cellulaire indifférenciée dont les cellules, après plusieurs repiquages, peuvent être transférées sur un milieu de culture. Après une dizaine de jours, la suspension cellulaire peut

2

être filtrée afin de ne retenir que les agrégats cellulaires de dimension désirée. Ces agrégats peuvent être cultivés sur un milieu de culture exempt d'hormone pendant quelques jours, afin d'induire la formation d'embryons.

Les embryons zygotiques peuvent être obtenus par prélèvement par dissection sur la graine au stade mature ou légèrement immature.

Les embryons somatiques et zygotiques obtenus peuvent être triés en fonction de leur stade de développement. On choisit de préférence les embryons aux premiers stades de leur développement, lorsqu'ils présentent une taille comprise entre environ 150 et 1000 $\mu$m. Ces dimensions correspondent aux stades de développement de type coeur ou de type torpille.

Les embryons obtenus peuvent ensuite être lavés, par exemple avec un milieu de culture liquide, usuel dans le domaine de la culture d'embryons, exempt d'hormones de croissance, tel qu'un milieu de Murashige et Skoog contenant 5 g/l de saccharose.

Les embryons lavés peuvent ensuite être transférés sur des plaques de culture, et séchés par aspiration du milieu de culture résiduel à l'aide d'une pipette par exemple.

Les embryons sont ensuite maintenus en hypoxie par enrobage avec une couche d'huile.

L'huile est choisie en fonction de son aptitude à provoquer l'hypoxie, c'est-à-dire à ne pas ou peu transférer l'oxygène extérieur vers l'embryon.

L'huile joue le rôle d'agent de conservation et fournit à l'embryon la quantité minimale d'oxygène requise pour assurer sa survie.

L'huile utilisée pour maintenir l'hypoxie peut être une huile d'origine organique ou végétale, une huile de synthèse ou toute autre huile susceptible d'assurer le maintien de l'hypoxie sans s'avérer toxique vis à vis des embryons.

L'huile utilisée est, de préférence, une huile de paraffine liquide.

L'huile peut préalablement avoir été dégazée et/ou stérilisée, par exemple par autoclavage à 115°C pendant 20 minutes.

La quantité d'huile ajoutée doit être suffisante pour assurer un enrobage complet des embryons. On ajoute, de préférence, environ 0,02 à 0,5 ml d'huile par embryon.

Les embryons sont ensuite refroidis jusqu'à une température légèrement supérieure au seuil de sensibilité au froid des embryons considérés.

Par seuil de sensibilité au froid, on entend la température en-dessous de laquelle les embryons ne sont plus viables.

La température à laquelle les embryons sont refroidis, puis conservés peut être supérieure de quelques degrés, de préférence de 1 à 10°C, au seuil de sensibilité au froid.

La température de conservation peut être, par exemple, comprise entre 2 et 8°C, de préférence 3 et 5°C, pour des embryons d'espèces peu sensibles au froid, telles que la carotte.

Elle peut être comprise entre 12 et 20°C, de préférence 15 et 17°C, pour des embryons d'espèces plus sensibles au froid, par exemple des espèces d'origine tropicale, telles que le caféier.

Le refroidissement des embryons peut s'effectuer en transférant par exemple, les plaques de culture contenant les embryons dans un réfrigérateur ou une enceinte climatisée.

La vitesse de refroidissement peut être assez rapide, de l'ordre de 1 à 3°C par minute par exemple.

Les embryons en hypoxie sous huile peuvent être conservés sous en éclairement faible, de l'ordre de 200 lux, ou dans l'obscurité.

Le présent procédé de conservation permet en effet d'assurer la survie d'embryons conservés à l'obscurité. Ceci peut présenter un avantage pratique, dans le cas où l'on réalise ultérieurement une semence artificielle par encapsulation de l'embryon. Il semble vraisemblable, en effet, qu'aucun éclairement résiduel ne parvienne à l'embryon à l'intérieur de la capsule.

Les embryons peuvent être conservés pendant d'assez longues périodes dans ces conditions, à savoir durant environ deux à quatre mois.

Après conservation, les embryons peuvent être sortis du réfrigérateur et réchauffés jusqu'à température ambiante. Lorsqu'ils ont atteint une température de l'ordre de 20°C, ils peuvent être lavés avec un milieu de culture liquide usuel afin d'éliminer toute trace d'huile.

Ils peuvent ensuite être placés dans ou sur un milieu de culture usuel, tel qu'un milieu de Murashige et Skoog, où ils reprennent un développement normal, comparable à celui d'embryons n'ayant pas subi de conservation.

Afin de permettre une diffusion auprès de l'utilisateur en vue d'un semis classique en pépinière ou en champ, il est possible d'encapsuler les embryons dans des matériaux plus résistants, qui leur offrent une protection comparable à celle des graines naturelles.

Dans ce cas, les embryons enrobés d'huile peuvent être encapsulés dans des polymères naturels ou artificiels, par exemple, un gel d'alginate de sodium. Ces capsules permettent d'assurer la protection mécanique et sanitaire de l'embryon, ainsi que l'alimentation de la plantule lors de sa germination. Les capsules peuvent être ultérieurement recouvertes d'une pellicule supplémentaire, formée par exemple d'un film de résine hydrosoluble, qui les préserve en partie de la rupture et du dessèchement. On obtient alors une semence artificielle qui peut se conserver pendant de plus longues périodes, à savoir la durée nécessaire à leur utilisation pratique.

La présente invention est illustrée plus en détail dans les exemples ci-après.

Ces exemples sont précédés par un exemple de préparation traditionnelle d'embryons somatiques, par la description d'un test de viabilité et par le tableau 1 qui donne la composition du milieu de culture préféré utilisé.

## Exemple de préparation d'embryons somatiques

Une suspension cellulaire indifférenciée de cellules de carottes Daucus carota L. est repiquée tous les 12 jours, à raison de 1 gramme de biomasse pour 100 ml de milieu, dans un milieu de culture liquide de Murashige et Skoog contenant 20 g/l de saccharose et 0,1 mg/l d'acide 2,4 dichlorophénoxyacétique.

Toutes les manipulations sont effectuées sous hotte à flux laminaire, dans des conditions aseptiques.

La suspension est placée sur un agitateur animé d'un mouvement giratoire excentré de 100 tours par minute, et est cultivée à 23°C, sous un éclairement de 200 lux avec une photopériode de 16 heures.

Après 8 à 10 jours de culture, la suspension cellulaire est filtrée afin de ne retenir que les agrégats cellulaires ayant une taille comprise entre 50 et 200 $\mu$m. Ces agrégats de faible taille représentent un stade proembryonnaire des embryons qui poursuivront leur développement jusqu'aux stades coeur, torpille et plantule. Les agrégats sont lavés et placés dans un milieu de Murashige et Skoog ne contenant pas d'acide 2,4 dichlorophénoxyacétique, à raison de environ $1,5.10^3$ agrégats par ml de milieu.

Après 10 jours de culture, des embryons se sont formés. On filtre la suspension de manière à ne retenir que les embryons ayant une taille comprise entre 150 et 1000 $\mu$m.

## Test de viabilité

Un test de viabilité, rapide et simple dans sa mise en oeuvre, a été établi afin d'évaluer le taux de viabilité des embryons après conservation.

Parmi les différents critères utilisables pour apprécier le taux de viabilité des embryons, on retient principalement:
- l'augmentation de la taille des embryons et
- l'apparition d'une coloration chlorophyllienne.

L'appréciation de ces critères peut être effectuée de différentes manières, par exemple par dénombrement visuel ou à l'aide de tests biochimiques (test de coloration par exemple).

Selon le principe de ce test, les embryons sont placés dans un milieu de culture liquide ou semi-solide usuel. On relève après 10 jours de culture, le nombre d'embryons dont la taille a augmenté et présentant des signes de coloration chlorophyllienne.

Le rapport de ce nombre au nombre total d'embryons présents permet de déterminer le taux de viabilité des embryons.

Les embryons peuvent ensuite être gardés sur le même milieu liquide ou placés sur un milieu de culture solide de même composition que le milieu liquide précédent afin de poursuivre leur développement vers le stade plantule. Après 10 jours de culture sur ce milieu, on détermine le taux de conversion comme étant le rapport entre le nombre d'embryons ayant évolués vers le stade plantule au nombre total d'embryons.

4

Tableau 1

| Composition du milieu de Murashige et Skoog (pH 5,8-6) | |
|---|---|
| Macroéléments | mg l$^{-1}$ |
| $NH_4NO_3$ | 1650 |
| $CaCl_2,2H_2O$ | 440 |
| $MgSO_4,7H_2O$ | 370 |
| $KNO_3$ | 1900 |
| $KH_2PO_4$ | 170 |
| Microéléments | |
| $CoCl_2$ | 0,025 |
| $CuSO_4,5H_2O$ | 0,025 |
| $FeSO_4,7H_2O$ | 27,8 |
| $Na_2$-EDTA | 37,3 |
| $MnSO_4,4H_2O$ | 22,3 |
| KI | 0,83 |
| $Na_2MoO_4$ | 0,25 |
| $ZnSO_4,7H_2O$ | 10,6 |
| $H_3BO_3$ | 6,2 |
| Autres éléments | |
| Acide nicotinique | 5 |
| Thiamine (vit.$B_1$) | 5 |
| Adénine | 2 |
| Saccharose | 5'000 |

Exemple 1

Des embryons somatiques de carotte, obtenus comme décrit ci-dessus, au stade torpille, d'une taille moyenne de 670 $\mu$m, sont lavés avec un milieu de culture liquide de Murashige et Skoog.

Les embryons sont ensuite placés sur des plaques de culture constituées de 6 puits, à raison de 20/30 embryons par puits, et débarrassés des traces de milieu de culture résiduel par aspiration du liquide avec une pipette.

Deux lots (A) et (B) d'embryons sont enrobés avec une couche d'huile de paraffine liquide, préalablement stérilisée par autoclavage à 115°C pendant 20 minutes. On ajoute environ 1 ml d'huile pour 5 à 8 embryons. Deux lots témoins (C) et (D) d'embryons sont enrobés avec le milieu de culture liquide.

Les plaques de culture sont recouvertes de leur couvercle et scellées hermétiquement.

Un premier lot (A) d'embryons en hypoxie sous huile est placé dans une enceinte réfrigérée à 4°C.

Pour comparaison, un second lot(B) d'embryons en hypoxie sous huile est placé à 23°C.

Un premier lot témoin (C) d'embryons n'étant pas en hypoxie est placé à 4°C, un second lot (D) est placé à 23°C.

Les différents lots sont conservés sous un éclairement de 200 lux.

Après un certain temps de conservation, les embryons sont réchauffés jusqu'à température ambiante puis on injecte du milieu de culture liquide sous la couche d'huile enrobant les embryons en hypoxie, afin de transférer le plus d'embryons possible dans le milieu de culture.

Après élimination de la couche d'huile, les embryons sont lavés dans plusieurs bains successifs de milieu de culture afin d'éliminer toute trace d'huile résiduelle.

On observe l'évolution de la taille des embryons au cours de la période de conservation, par mesure de leur taille à l'aide d'une loupe binoculaire équipée d'un oculaire de mesure.

On obtient les résultats suivants:

| Taille des embryons ($\mu$m) | | | | | | |
|---|---|---|---|---|---|---|
| | Temps de conservation (jours) | | | | | |
| | 0 | 14 | 35 | 49 | 63 | 105 |
| Lot A (4°C) | 670 | 674 | 732 | 762 | 854 | 1327 |
| Lot B (23°C) | 670 | 2863 | 2896 | -- | -- | -- |
| Témoin (4°C) | 670 | 688 | 1104 | 1292 | 1536 | 3310 |
| Témoin (23°C) | 670 | 10500 | -- | -- | -- | -- |

On remarque que les embryons conservés a 23°C croissent assez rapidement, qu'ils soient ou non en hypoxie. Après 14 jours, leur taille a au moins triplé, alors que les embryons conservés à 4°C n'ont presque pas évolué. Après 105 jours, les embryons conservés à 4°C en hypoxie sous huile présentent encore une taille relativement faible, alors que les embryons témoins conservés à 4°C en milieu de culture ont continué de croître.

Parallèlement, on observe l'influence de la conservation en hypoxie sous huile à 4°C sur la viabilité des embryons.

A cet effet, les embryons débarrassés de la couche d'huile et réchauffés jusqu'à température ambiante sont mis en culture dans le milieu de culture liquide de Murashige et Skoog.

La viabilité des embryons est appréciée par la reprise ou la poursuite de leur développement en milieu liquide.

Après 10 jours de culture, on relève le taux de viabilité des embryons.

On obtient les résultats suivants:

| Taux de viabilité: (exprimé en % de reprise de croissance) | | | | | | |
|---|---|---|---|---|---|---|
| | Temps de conservation (jours) | | | | | |
| | 0 | 14 | 35 | 49 | 63 | 105 |
| lot A (4°C) | 97 | 94 | 97 | 92 | 90 | 65 |
| lot B (23°C) | 97 | 96 | 18 | 0 | -- | -- |
| Témoin (4°C) | 97 | 92 | 92 | 91 | 87 | 83 |
| Témoin (23°C) | 97 | 90 | -- | -- | -- | -- |

Les embryons conservés en hypoxie sous huile à 4°C présentent un taux de viabilité correct après 105 jours de conservation, alors que les embryons conservés en hypoxie à 23°C ne sont plus viables après une durée de conservation de 35 jours.

Les embryons témoins conservés à 4°C présentent aussi une bonne viabilité après 105 jours, mais leur taille est alors trop importante (environ 3300 $\mu$m) pour permettre une éventuelle conservation par encapsulation dans un polymère.

Après 20 jours de culture dans le milieu liquide, on relève le nombre d'embryons ayant développés une plantule normale.

On obtient les résultats suivants:

| Taux de conversion (exprimé en % de plantules développées) | | | | |
|---|---|---|---|---|
| | Temps de conservation (jours) | | | |
| | 0 | 35 | 63 | 105 |
| Lot A (4°C) | 97 | 99 | 80 | 36 |
| Témoin (4°C) | 97 | 86 | 23 | 0 |

Les embryons ayant été conservés à 4°C en hypoxie sous huile continuent leur croissance, sans apparition de prolifération adventive, de manière identique à celle des embryons témoins.

L'inhibition partielle de la croissance des embryons somatiques par maintien en hypoxie sous huile à 4°C n'a pas affecté la viabilité ou le pouvoir de reprise de développement des embryons.

Exemple 2

Des embryons somatiques de carotte, de taille moyenne de 460 $\mu$m sont conservés à 4°C selon la méthode décrite à l'exemple 1.

Une partie des embryons est conservée à l'obscurité et l'autre partie est conservée, pour comparaison, sous 200 lux.

On observe l'évolution de la taille des embryons au cours de la période de conservation.

On obtient les résultats suivants:

| Taille des embryons ($\mu$m) | | | | | | | |
|---|---|---|---|---|---|---|---|
| | Temps de conservation (jours) | | | | | | |
| | 0 | 18 | 29 | 46 | 61 | 81 | 95 |
| Hypoxie sous huile obscurité | 460 | 596 | 601 | 701 | 625 | 545 | 554 |
| Hypoxie sous huile 200 lux | 460 | 610 | 603 | 659 | 547 | 651 | 548 |
| Témoin obscurité | 460 | 999 | 1325 | 2811 | 3644 | 7454 | 11670 |
| Témoin 200 lux | 460 | 1120 | 1508 | 2493 | 4634 | 7602 | 9941 |

On remarque que les embryons conservés en hypoxie sous huile n'ont presque pas évolué en taille, qu'ils aient été conservés à la lumière ou dans l'obscurité.

L'éclairement de l'embryon lors de sa conservation ne semble pas influencer sa viabilité.

L'absence de lumière n'a pas d'incidence sur le degré d'inhibition induit par la combinaison de l'hypoxie sous huile et d'une température basse.

Exemple 3

Des embryons somatiques de caféier Coffea arabica au stade torpille avancée, d'une taille moyenne de 1590 $\mu$m sont lavés et placés sur des plaques de culture selon la méthode décrite à l'exemple 1.

Trois lots (A), (B) et (C) d'embryons sont enrobés avec une couche d'huile de paraffine liquide stérilisée, à raison de environ 1 ml d'huile pour 5 à 8 embryons. Trois lots témoins d'embryons sont enrobés avec un milieu liquide de Murashige et Skoog.

Un lot (A) d'embryons en hypoxie sous huile est placé à 4°C, un second lot (B) à 10°C et un troisième lot (c) à 15°C.

Un premier lot témoin est placé à 4°C, un second à 10°C et un troisième à 15°C.

Les lots sont conservés à l'obscurité pendant un mois puis sont ramenés à température ambiante.

Les embryons sont lavés selon la méthode décrite à l'exemple 1, puis mis en culture sur un milieu semi-solide de Murashige et Skoog.

Après 10 jours de culture, on relève le taux de viabilité des embryons.

On obtient les résultats suivants:

## Taux de viabilité (% de reprise de croissance)

| | | |
|---|---|---|
| Lot A (4°C) | 0 | } comparaison |
| Lot B (10°C) | 0 | |
| Lot C (15°C) | 80 | |
| Témoin (4°C) | 0 | } comparaison |
| Témoin (10°C) | 0 | |
| Témoin (15°C) | 84 | |

On remarque que les embryons conservés à 4°c ou 10°C brunissent et meurent, qu'ils soient en hypoxie sous huile ou dans un milieu de culture.

Les embryons de caféier se conservent bien à une température de 15°C.

Il est donc important de prendre en compte le seuil de sensibilité au froid de l'espèce considérée afin de déterminer la température minimale de conservation.

Exemple 4

Des embryons somatiques de caféier au stade torpille avancé, d'une taille moyenne de 1590 $\mu$m sont conservés à 15°C en hypoxie sous huile selon la méthode décrite à l'exemple 1.

Après 1 ou 2 mois de conservation, les embryons sont réchauffés jusqu'à température ambiante et lavés.

On observe l'évolution de la taille des embryons au cours de la période de conservation.

On obtient les résultats suivants :

| Taille des embryons ($\mu$m) | | | |
|---|---|---|---|
| | Temps de conservation | | |
| | 0 | 1 mois | 2 mois |
| Embryons en hypoxie sous huile | 1590 | 1680 | 1710 |
| Embryons témoins | 1590 | 2780 | 3590 |

On remarque que les embryons en hypoxie sous huile croissent beaucoup plus lentement que les embryons témoins.

On relève, après 10 jours de culture des embryons sur milieu semi-solide, le taux de viabilité.

Il est de 70% pour les embryons conservés 2 mois en hypoxie sous huile et de 84% pour les embryons témoins conservés 2 mois dans le milieu de culture liquide. L'enrobage des embryons avec une couche d'huile permet donc bien d'inhiber leur croissance sans altérer sérieusement leur viabilité.

Exemple 5

Des embryons somatiques de caféier, au stade coeur avancé, d'une taille moyenne de 1100 $\mu$m ou au stade torpille, d'une taille moyenne de 1320 $\mu$m, sont conservés à l'obscurité à 15°C pendant 1 mois, soit en hypoxie sous huile de paraffine, soit en milieu de culture liquide, selon la méthode décrite à l'exemple 1.

Après conservation, les embryons sont réchauffés jusqu'à température ambiante et lavés.

On observe l'évolution de la taille des embryons au cours de la période de conservation.

On obtient les résultats suivants:

| Taille des embryons ($\mu$m) | | |
|---|---|---|
| | Temps de conservation | |
| | 0 | 1 mois |
| Embryons coeur avancé | | |
| Hypoxie sous huile | 1'100 | 1'300 |
| Témoin | 1'100 | 2'470 |
| Embryons torpille | | |
| Hypoxie sous huile | 1'320 | 1'750 |
| Témoin | 1'320 | 2'790 |

De la même manière que dans l'exemple 4, on remarque que les embryons en hypoxie sous huile croissent plus lentement que les embryons témoins.

On cultive pendant 10 jours les embryons sur un milieu semi-solide de Murashige et Skoog, et on relève le taux de viabilité.

On obtient les résultats suivants:

**EP 0 408 922 B1**

| Embryons coeur avancé | Taux de viabilité (%) |
|---|---|
| - Hypoxie sous huile<br>- Témoin | 76<br>84 |
| Embryons torpille | |
| - Hypoxie sous huile<br>- Témoin | 71<br>95 |

## Revendications

1. Procédé de conservation d'embryons végétaux, caractérisé par le fait que les embryons sont maintenus en hypoxie par enrobage avec une couche d'huile puis refroidis et conservés à une température supérieure au seuil de sensibilité au froid des embryons considérés.

2. Procédé selon la revendication 1, caractérisé par le fait que les embryons sont des embryons somatiques.

3. Procédé selon la revendication 1, caractérisé par le fait que l'huile maintenant l'hypoxie est une huile d'origine organique ou végétale ou une huile de synthèse.

4. Procédé selon la revendication 3, caractérisé par le fait que l'huile maintenant l'hypoxie est une huile de paraffine liquide.

5. Procédé selon la revendication 1, caractérisé par le fait que les embryons en hypoxie sous huile sont conservés à une température supérieure de 1 à 10°C au seuil de sensibilité au froid des embryons considérés.

6. Procédé selon la revendication 1, caractérisé par le fait que les embryons sont conservés à l'obscurité.

7. Procédé selon la revendication 1, caractérisé par le fait que les embryons sont des embryons somatiques de carotte et qu'ils sont conservés à une température comprise entre 2 et 8°C.

8. Procédé selon la revendication 1, caractérisé par le fait que les embryons sont des embryons somatiques de caféier et qu'ils sont conservés à une température comprise entre 12 et 20°C.

9. Procédé de conservation d'embryons végétaux, caractérisé par le fait que les embryons enrobés d'huile sont encapsulés dans des polymères naturels ou artificiels.

10. Semence artificielle obtenue selon la revendication 9.

## Claims

1. A process for storing plant embryos, characterized in that the embryos are kept under hypoxia by coating with a layer of oil and then cooled and stored at a temperature above the cold sensitivity threshold of the embryos in question.

2. A process as claimed in claim 1, characterized in that the embryos are somatic embryos.

3. A process as claimed in claim 1, characterized in that the oil maintaining hypoxia is a mineral oil, an oil of vegetable origin or a synthetic oil.

4. A process as claimed in claim 3, characterized in that the oil maintaining hypoxia is a liquid paraffin oil.

5. A process as claimed in claim 1, characterized in that the embryos under hypoxia in oil are stored at a temperature 1 to 10°C above the cold sensitivity threshold of the embryos in question.

9

**6.** A process as claimed in claim 1, characterized in that the embryos are stored in darkness.

**7.** A process as claimed in claim 1, characterized in that the enbryos are somatic carrot embryos and are stored at a temperature of 2 to 8°C.

**8.** A process as claimed in claim 1, characterized in that the embryos are somatic coffee tree embryos and are stored at a temperature of 12 to 20°C.

**9.** A process for storing plant embryos, characterized in that the oil-coated embryos are encapsulated in natural or artificial polymers.

**10.** Artificial seeds obtained in accordance with claim 9.

**Patentansprüche**

**1.** Verfahren zur Konservierung pflanzlicher Embryonen, dadurch gekennzeichnet, daß die Embryonen durch Umhüllung mit einem Ölbett unter Hypoxie gehalten werden und dann abgekühlt und bei einer Temperatur oberhalb der Empfindlichkeitsschwelle der jeweiligen Embryonen gegenüber Kälte konserviert werden.

**2.** Verfahren nach Anspruch 1, dadurch gekennzeichnet, daß die Embryonen somatische Embryonen sind.

**3.** Verfahren nach Anspruch 1, dadurch gekennzeichnet, daß das Öl zur Aufrechterhaltung der Hypoxie ein Öl organischen oder pflanzlichen Ursprungs oder ein synthetisches Öl ist.

**4.** Verfahren nach Anspruch 3, dadurch gekennzeichnet, daß das Öl zur Aufrechterhaltung der Hypoxie ein flüssiges Paraffinöl ist.

**5.** Verfahren nach Anspruch 1, dadurch gekennzeichnet, daß die Embryonen unter Hypoxie unter Öl bei einer Temperatur konserviert werden, die 1 bis 10°C oberhalb der Empfindlichkeitsschwelle der jeweiligen Embryonen gegenüber Kälte liegt.

**6.** Verfahren nach Anspruch 1, dadurch gekennzeichnet, daß die Embryonen in der Dunkelheit konserviert werden.

**7.** Verfahren nach Anspruch 1, dadurch gekennzeichnet, daß die Embryonen somatische Karottenembryonen sind und bei einer Temperatur zwischen 2 und 8°C konserviert werden.

**8.** Verfahren nach Anspruch 1, dadurch gekennzeichnet, daß die Embryonen somatische Kaffeebaumembryonen sind und bei einer Temperatur zwischen 12 und 20°C konserviert werden.

**9.** Verfahren zum Konservieren pflanzlicher Embryonen, dadurch gekennzeichnet, daß die mit Öl umhüllten Embryonen in natürliche oder künstliche Polymere verkapselt werden.

**10.** Ein künstlicher Samen, der nach Anspruch 9 erhalten wurde.